# EUROPEAN PATENT APPLICATION

(11) **EP 1 522 320 A1**
(43) Date of publication of application: **13.04.2005**
(21) Application number: 04256215.7
(22) Date of filing: 08.10.2004
(51) Int. Cl.: A61L 9/12, A01M 1/20

(54) **Fan-driven air freshener**

(30) Priority: 09.10.2003 US 682051
(71) Applicant: INTERNATIONAL FLAVORS & FRAGRANCES INC., New York New York 10019 (US)
(72) Inventor: Selander, Raymond K., Hopewell Junction, New York 12533 (US)
(74) Representative: Mercer, Christopher Paul

(57) **Abstract**

The present invention combines an air freshener (100) that has a source (50) of air freshening chemical with a fan (120) that is controlled either by an optical device (110) that senses light or a motion detector. When a light is turned on or motion is detected, the fan (120) will be activated for a predetermined time period. In certain embodiments, the fan (120) will stop turning after a predetermined time. Alternatively, the fan (120) can continue to be powered until the light source is turned off, or all motion ceases, and only then either immediately shut down, or shut down after a predetermined time period. In certain preferred embodiments the source (50) of air freshening chemical is disposed beneath the fan and allows fragrance to be delivered over time without the fan (120). The additional airflow provided by the fan (120) causes more volatile fragrance chemicals to be removed from the source of air freshening chemical and admitted into the environment. The fan motor (124) of the present invention is driven by a power source (130), such as batteries, AC line current or alternate sources such as solar cells. Preferably, a microprocessor controls the fan (120) so that a "burst mode" is created by controlling the frequency and intensity of the pulses of air freshener that are emitted.

## Description

The present invention relates to fragrance delivery systems, and more particularly to active systems in which a fan suffuses the air in an environment with a chemical to mask or minimize objectionable odors.

### BACKGROUND OF THE INVENTION

Various devices are known that "freshen" air by adding a fragrance chemical to the air. In particular, off odors and malodors found in bathrooms are common. Various devices and chemicals that either disinfect, i.e., kill odor causing bacteria, or spray a perfume or fragrance to mask odors are known. Although many of these systems are passive and emit an air freshening compound into the air continuously, others use a fan to circulate the air freshening compound more rapidly and in higher concentration.

Currently available air fresheners with fans have various limitations. One limitation is that they do not deliver air freshening compounds effectively, primarily because the compound is delivered in intermittent bursts of varying intensity, or pulses, while the fan is operating. Additionally, currently available designs simply turn the fan on and off manually. If the fan is activated for a period of time beyond that needed the life of the fan and motor assembly is shortened unnecessarily, as is the battery life in battery-driven models. Moreover, air freshening chemicals volatilized by the fan are used up more quickly if the fan is either constantly running or running for a period of time longer than necessary.

U.S. Patent No. 4,695,435 (Spector) discloses an air freshener device with a motor driven fan that is activated by a light being turned on, and is deactivated when the light is turned off.

U.S. Patent No. 4,707,338 (Spector) discloses an air freshener device with a motor driven fan that is activated by a light being turned on, and is deactivated after a set period of time.

Neither of these prior art devices address the problems outlined above. Therefore, there remains a long-felt yet unmet need for providing enhanced levels of volatile air freshening or aroma chemicals in an effective and efficient manner. It would therefore be desirable to provide materials and methods that enhance the efficiency of fan driven air freshening systems. It would further be desirable to provide such improvements in a manner that permitted their application across a wide variety of situations and that permitted their implementation in a cost-effective manner.

### SUMMARY OF THE INVENTION

Accordingly, it has now been found that these and other problems found in the prior art can be overcome by an air freshener apparatus that has a source of air freshening chemical, a photocell and a fan assembly disposed in a housing adjacent the source of air freshening chemical. The fan is controlled by the optical sensor such that the fan motor is activated for a predetermined time period upon the photocell sensing a predetermined level of light. In preferred embodiments, the source of air freshening chemical is a wick, and most preferably, the wick is disposed beneath the fan and allows fragrance to be delivered over time without the fan. In certain embodiments, the air freshener also has a control circuit, or shutoff circuit that deactivates the fan motor after a predetermined time, or alternatively shuts the motor off if the sensor senses a level of light below a predetermined level, either immediately or after a predetermined length of time. The fan motor is either driven by direct current or AC line current. In the latter, in certain preferred embodiments, the housing comprises a plug that connects the motor to the AC line current via a wall outlet and a receptacle wherein the wall outlet retains its utility and can be used to power another device simultaneously with the fan.

In one aspect of certain preferred embodiments of the present invention, a microprocessor is connected to the fan motor, and drives the fan at a predetermined frequency for a predetermined duration. Most preferably, the microprocessor is connected to a micropump and to an electro spray device.

In alternate embodiments, the air freshener apparatus uses a motion sensor to control the fan. In these embodiments, the fan motor is activated for a predetermined time period upon the motion sensor being activated, and the device also has a shutoff circuit. In a manner similar to the optical sensor embodiments, the shutoff circuit either deactivates the fan motor after a predetermined time which is either pre-set or determined by the absence of motion.

### BRIEF DESCRIPTION OF THE DRAWINGS

**Fig. 1** is a side elevation view of a first embodiment of a fan driven air freshener made in accordance with the present invention.
**Fig. 2** is a perspective view of a second embodiment of a fan driven air freshener made in accordance with the present invention.

### DETAILED DESCRIPTION OF THE PREFERRED EMBODIMENTS

The implementation of the present invention is in several preferred embodiments, discussed below, along with several illustrative examples. The embodiments of the invention described below are provided for the purpose of understanding the invention and are not meant to be limiting.

The present invention is well suited for the delivery of fragrance chemicals as well as those materials that work to minimize or block malodor. These air freshening materials can be used in the present invention as liquids, as concentrated oils, encapsulated particles, as a gel, or other forms that can be incorporated into the present invention.

Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, and carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrance materials is provided in U.S. Patent No. 4,534,891. Another source of suitable fragrances is found in Perfumes, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like. The above list is but a description of fragrances that can be used in the present invention.

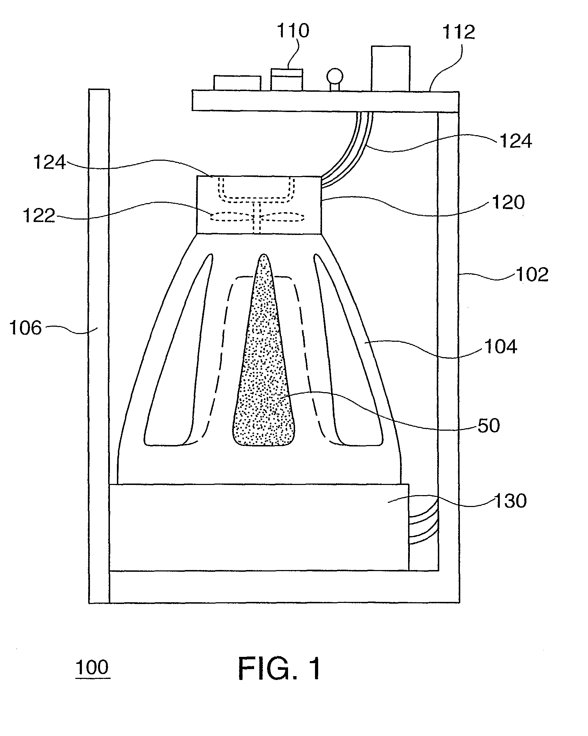

Referring now to FIG. 1, a side elevation view of a preferred embodiment of a fan driven air freshener 100 made in accordance with the present invention is illustrated. The apparatus is contained within a housing 102. In order to illustrate the invention those of skill in the art will understand that one side panel of the housing 102 has been removed. It will be further understood that the housing can be any of a number of designs and shapes, and is not limited to that which is illustrated. Generally, the housing 102 may be constructed from metal, plastic or any other suitable material that has sufficient structural strength to hold the components as shown, while permitting sufficient airflow and that meets any safety or aesthetic criteria. Typically, but not necessarily, at least a portion of the housing 102 will comprise an air permeable panel 106 through which air may intermingle with air freshening chemicals. The chemicals are held in a reservoir 50, such as a wick, as is well known in the art. The chemicals may be any combination of odor masking or odor eliminating compounds that react with malodors or that have a more pleasant aroma than malodors. The composition and concentration of such chemicals for this use is well known. In a preferred embodiment, the reservoir 50 is contained within a shroud or reservoir housing 104. Preferably, the reservoir housing 104 allows the reservoir 50 to be handled without spilling or degrading the chemicals, and in certain embodiments may permit the chemicals to be replaced after they have dissipated by replacing the reservoir housing 104 and the reservoir 50 that contains fresh chemicals.

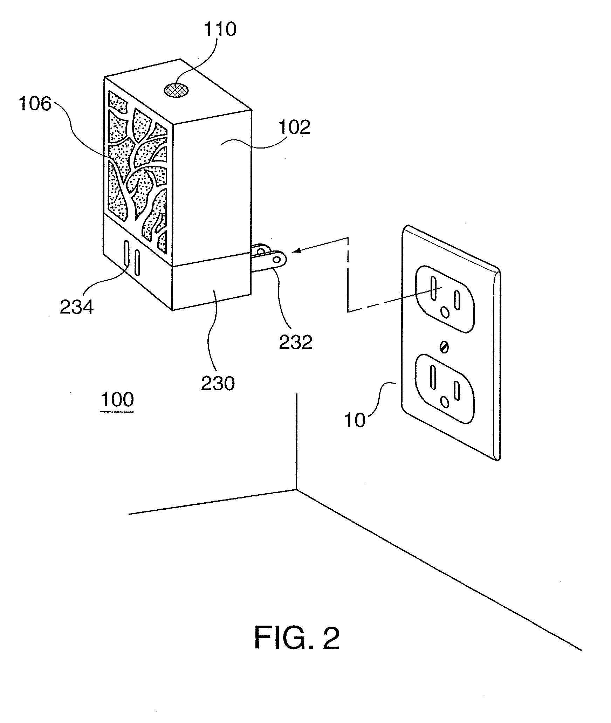

As illustrated in FIG. 1, a fan assembly 120 is preferably disposed above the reservoir 50 so as to force air through the apparatus. In certain preferred embodiments, placing the fan 120 over the reservoir 50 is preferred and is more effective than placing the fan 120 adjacent the reservoir 50. Moreover, such an embodiment can be constructed by modifying an existing air freshener assembly, which is less expensive than creating an entirely new assembly that positions the components elsewhere. The fan assembly 120 typically comprises a rotor 122 and a fan motor 124. Preferably the fan is positioned in the horizontal plane disposed above the reservoir. Miniature fans suitable for any number of various embodiments of the present invention are readily available and easily adapted to the configuration shown in FIG. 1. The fan assembly 120 is driven by a power source 130. In the embodiment shown in FIG. 1 the power source 130 is preferably a direct current source, such as a battery. In addition to batteries, other conventional direct current power sources, such as solar cells, for one example, may be included in other embodiments. However, as explained in further detail below with reference to FIG. 2, the present invention also contemplates embodiments that use alternating current. The power source 130 is connected to a control circuit 112 by wires 124. As explained in further detail below, the control circuit 112 determines when the fan motor 124 is activated, and the duration of its activation.

In certain embodiments of the present invention, the control circuit 112 includes a sensor or photocell 110 that senses the level of light in the environment, and activates or deactivates a switch that supplies power to the fan motor 124. For example, the cell 110 can be chosen and put into a circuit so that the fan motor 124 is activated when a light is turned on in the room in which the apparatus is positioned. The control circuit 112 can also provide controls so that the fan 120 runs until the light is shut off, and then deactivates immediately. Alternatively, the fan 120 could run for a predetermined time (e.g., five minutes) or for a fixed time after the light source changes again, for example, when a light is turned off. The selection of a photocell and the components of the control circuit is conventional and well within the level of skill in the art. By running the fan motor 124 only when necessary, the component life is extended and the fragrance materials in the reservoir 50 are preserved.

Alternatively, in certain other preferred embodiments, the photocell 110 is replaced by a motion detector 110. In much the same manner as described in the preceding paragraph, the motion detector 110 determines when the fan assembly 120 should be activated, and in conjunction with the control circuit 112 determines how long a period of time the fan rotor 122 will turn. As mentioned above, the fan 120 is activated only when motion is sensed and shut off immediately in the absence of motion. Alternatively, the fan 120 can be activated when motion is sensed and then run for a fixed period. Finally, the fan can be activated and then run for a period of time measured after all motion has ceased. The selection of a motion detector and the components of the control circuit is conventional and well within the level of skill in the art.

Referring now to FIG. 2, an alternate embodiment of the present invention is illustrated. In this embodiment AC line current is used as a power source. As shown, the air freshener 100 contains an AC power supply 230 necessary to convert the line current and provide safety, if required, via a ground fault interrupter or similar circuit. The air freshener 100 has conventional plug prongs 232 that connect to a conventional receptacle 10. In the preferred embodiment illustrated in FIG. 2, a receptacle 234 is provided that is part of the power supply assembly 230 and plug prongs 232 that connects the assembly to the power source, so that the AC power outlet retains its utility and can be used to power another device simultaneously with the fan. Alternatively, in certain embodiments, particularly those intended for non-home use, the air freshener 100 is connected directly to an AC source via a junction box or similar wiring device and is thus permanently installed in terms of the electrical connection. The alternating current embodiment illustrated in FIG. 2 is otherwise identical to that described above with reference to FIG. 1.

In accordance with on aspect of the present invention, a "burst" mode of operation is provided. It has been found that by providing a microprocessor to control the operation of the fan described above, dramatic improvement in performance can be attained. In a most preferred embodiment, the flexibility of programming a microprocessor is utilized to its fullest advantage by incorporating a micro pump into the reservoir described above and driving the pump at a first frequency, and simultaneously driving an atomizing device such as an electro sprayer at a second frequency. The selection of ideal frequencies for any particular fragrance chemical combination is routine and does not require undue experimentation. However, in any embodiment, air freshener chemical will be introduced into the air even when the fan is deactivated. Experiments have shown that adding a burst mode to the above-described device can provide 2.8 times the evaporation (i.e., a 280 % increase) an effect particularly well-suited for bathrooms, where it is important to modify the air for short periods of time. Further improvement is the delivery of the air freshening materials are anticipated by producing various modifications of the apparatus described.

| **System Type** | **Time (hr.)** | **Start Wt. (g)** | **End Wt. (g)** | **Rate (g/hr.)** |
|---|---|---|---|---|
| Conventional | 16.2 | 217 | 215.6 | 0.00144 |
| Fan System: | 16.2 | 58.9 | 58.4 | 0.00052 |

Upon review of the foregoing, numerous adaptations, modifications, and alterations will occur to the reviewer. These will all be, however, within the spirit of the present invention. Accordingly, reference should be made to the appended claims in order to ascertain the true scope of the present invention.

## Claims

1. Air freshener apparatus comprising:
a source of air freshening chemical;
a photocell; and
a fan assembly comprising a fan and a fan motor disposed in a housing adjacent the source of air freshening chemical that is controlled by the optical sensor,
wherein the fan motor is activated for a predetermined time period upon the photocell sensing a predetermined level of light.

2. The apparatus of claim 1, further comprising a shutoff circuit, wherein the shutoff circuit immediately deactivates the fan motor when the photocell senses a level of light below a predetermined level.

3. Air freshener apparatus comprising:
a source of air freshening chemical;
a motion sensor; and
a fan assembly comprising a fan and a fan motor disposed in a housing adjacent the source of air freshening chemical that is controlled by the motion sensor,
wherein the fan motor is activated for a predetermined time period upon the motion sensor being activated.

4. The apparatus of any one of claims 1 to 3, wherein the source of air freshening chemical is a wick.

5. The apparatus of any one of claims 1 to 4, wherein the source of air freshening chemical is disposed beneath the fan and allows fragrance to be delivered over time without the fan.

6. The apparatus of any one of claims 1 to 5, further comprising a shutoff circuit, wherein the shutoff circuit deactivates the fan motor after a predetermined time.

7. The apparatus of claim 15, wherein the predetermined time is determined by whether photocell senses a level of light below a predetermined level or whether the motion sensor senses a level of motion below a predetermined level.

8. The apparatus of any one of claims 1 to 8, wherein the fan motor is driven by direct current.

9. The apparatus of any one of claims 1 to 8, wherein the fan motor is driven by AC line current.

10. The apparatus of claim 9, wherein the housing comprises a plug that connects the motor to the AC line current via a wall outlet and a receptacle wherein the wall outlet retains its utility and can be used to power another device simultaneously with the fan.

11. The apparatus of any one of claims 1 to 10, further comprising a microprocessor connected to the fan motor, whereby the microprocessor drives the fan at a predetermined frequency for a predetermined duration.

12. The apparatus of claim 11, wherein the microprocessor is connected to a micropump and to an electro spray device.
